# EUROPEAN PATENT SPECIFICATION

(11) **EP 2 505 120 B1**
(45) Date of publication and mention of the grant of the patent: **26.08.2015**
(21) Application number: 12157233.3
(22) Date of filing: 28.02.2012
(51) Int. Cl.: A61B 1/06, A61B 1/00, A61B 1/07

(54) **Endoscope and lighting optical device therefor**
Endoskop und optische Beleuchtungsvorrichtung
Endoscope et son dispositif optique d'éclairage

(30) Priority: 28.03.2011 JP 2011069499
(43) Date of publication of application: 03.10.2012
(60) Divisional application: 13166716.4
(73) Proprietor: Fujifilm Corporation, Minato-ku Tokyo (JP)
(72) Inventor: Kitano, Ryou, Kanagawa (JP)
(74) Representative: Klunker . Schmitt-Nilson . Hirsch

(56) References cited:
- JP-A- 2002 182 126
- JP-A- 2004 283 426
- US-A- 4 580 552
- US-A- 4 736 734
- US-A1- 2009 093 681

## Description

### BACKGROUND OF THE INVENTION

### 1. Field of the invention

The present invention relates to an endoscope according to the preamble of claim 1.

### 2. Description Related to the Prior Art

A medical electronic endoscope has an insert section to be inserted into a subject and a handling section for operating the insert section. A distal portion of the insert section incorporates an imaging optical device and a lighting optical device. An image of a target illuminated with the lighting optical device is captured with the imaging optical device. An observation image is displayed on a monitor or the like. A light guide extends throughout the insert section from its base portion to the distal portion. The light guide is, for example, a plurality of optical fibers in a bundle. The illumination light from an external light source device is incident on an end (incident end) and then comes out of the other end (exit end) of the light guide. Then the illumination light is applied to the target through the lighting optical device.

A lighting optical device using a rod-like optical element is disclosed in, for example, Japanese Patent Laid-Open Publication No. 2002-182126. This optical element has a core surrounded with a clad to obtain a suitable light distribution property. A refractive index of a clad is lower than that of the core. In this lighting optical device, two plano-convex lenses are disposed with an appropriate space therebetween on an exit side of the optical element. Light rays, parallel with an optical axis of the illumination light, coming out of the light guide are converged in the lighting optical device and then spread to achieve required light distribution.

Temperature rise of the distal portion of the electronic endoscope increases noise in an imaging signal from an image sensor. This degrades image quality and may also affect a subject. Measures have been taken to prevent the temperature rise in the distal portion. However, higher pixel density increases consumption power, which increases generation of heat in the distal portion.

To prevent the temperature rise of the distal portion, a heat ray cut filter is used, for example. The illumination light from the light source device passes through the heat ray cut filter before entering the light guide to cut infrared rays. However, light quantity loss, caused by blockage of a part of the illumination light inside the lighting optical device or the like, generates heat. This causes the temperature rise of the distal portion.

The illumination light coming out of the light guide includes light rays spread in accordance with a numerical aperture. In the lighting optical device of the Japanese Patent Laid-Open Publication No. 2002-182126, there are light rays traveling in directions non-parallel with the optical axis and then coming out, in directions approximately parallel with the optical axis, of a peripheral portion of an exit surface of the rod-like optical element. When a spacer ring is disposed between the optical element and a lens to determine the distance therebetween, the light rays coming out of the peripheral portion of the exit surface of the optical element and then traveling approximately parallel with the optical axis are blocked by the spacer ring. This blockage of the light rays results in the light quantity loss, which reduces illumination efficiency and increases temperature of the distal portion.

In accordance with the preamble of claim 1, US 4 736 734 A discloses an endoscope in which the optical element held inside a frame (a lens barrel) may be moved in the direction of its optical axis in order to vary the angle of view of the optical system.

### SUMMARY OF THE INVENTION

An object of the present invention is to provide an endoscope for reducing light quantity loss of illumination light.

To achieve the above and other objects, an endoscope includes the features of claim 1.

It is preferable that the lighting optical device further includes a light guide mounting hole, formed inside the distal portion body, for housing the light guide. The light guide mounting hole is connected to the lens mounting hole. An outer diameter of the light guide mounting hole is smaller than an outer diameter of the lens mounting hole.

It is preferable the outer diameter of the optical element is approximately equivalent to an outer diameter of the light guide.

It is preferable that the lighting optical device further includes a second lens disposed in front of the first lens inside the lens mounting hole, and an outer diameter of the second lens is equivalent to the outer diameter of the first lens.

It is preferable that the incident surface of the optical element is planar and approximately flush with a rear edge of the lens barrel.

It is preferable that the optical element is a rod lens including a core and a clad surrounding the core. A refractive index of the clad is lower than that of the core.

It is preferable that the endoscope further includes a spacer ring disposed between the first and second lenses to keep a distance therebetween.

It is preferable that the lens barrel is shorter than the lens mounting hole. A front edge of the lens barrel is positioned inside the lens mounting hole. The first lens contacts the front edge of the lens barrel. The first lens, the spacer ring, and the second lens fit into the lens mounting hole.

It is preferable that a length of the lens barrel is equivalent with a length of the lens mounting hole. It is preferable that the lens barrel has a small diameter portion, a large diameter portion, and a shoulder portion formed between the small and large diameter portions. The optical element is fitted into the small diameter portion. The first lens contacts the shoulder portion. The first lens, the spacer ring, and the second lens are fitted into the large diameter portion.

According to the present invention, a lens with the outer diameter greater than that of the optical element is disposed in front of the optical element. The optical element has the outer circumferential portion with the light reflection function and a convex exit surface. The diameter of the space between the optical element and the lens is greater than or equal to the outer diameter of the optical element. Accordingly, the light rays coming out of the peripheral portion of the exit surface of the optical element and then traveling approximately parallel with the optical axis are incident on the lens without obstruction. This reduces light quantity loss of the illumination light and prevents the temperature rise of the distal portion resulting from heat generation caused by the light quantity loss.

### BRIEF DESCRIPTION OF THE DRAWINGS

The above and other objects and advantages of the present invention will be more apparent from the following detailed description of the preferred embodiments when read in connection with the accompanied drawings, wherein like reference numerals designate like or corresponding parts throughout the several views, and wherein:
Fig. 1 is a schematic view of an endoscope system;
Fig. 2 is an exploded perspective view of a lighting optical device;
Fig. 3 is a cross section of the lighting optical device;
Fig. 4 is a cross section showing an optical element and first and second lenses, fitted in a lens barrel by way of example;
Fig. 5 is a cross section showing the optical element and first and second lenses, directly fitted in a lens mounting hole by way of example; and
Fig. 6 is a cross section of the lens barrel fixed with a screw.

### DESCRIPTION OF THE PREFERRED EMBODIMENTS

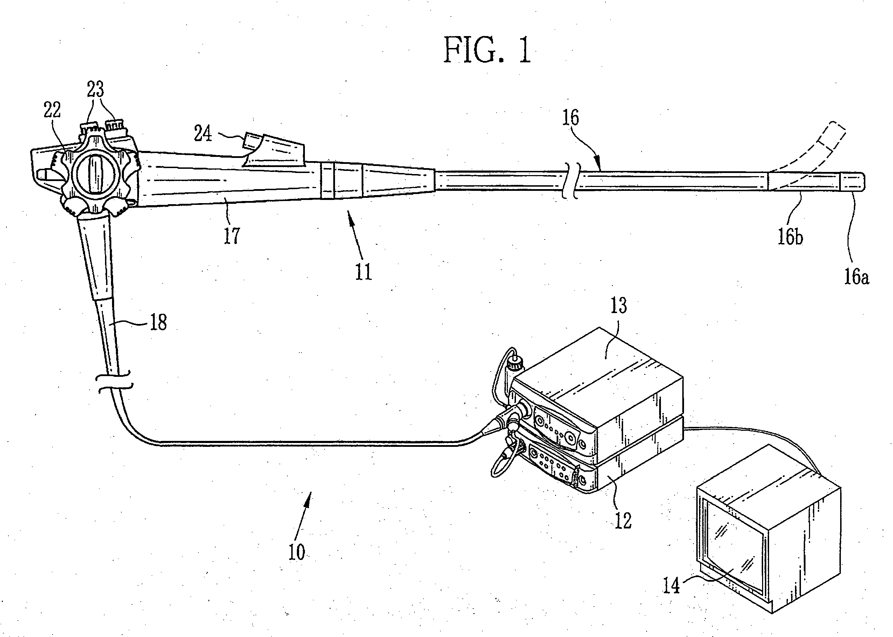

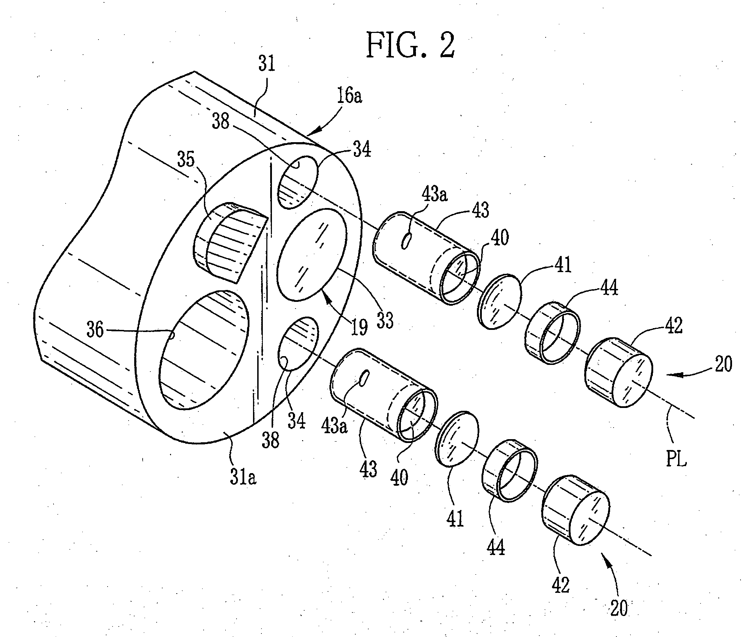

In Fig. 1, an endoscope system 10 is provided with an electronic endoscope 11, a processing device 12, and a light source device 13. The electronic endoscope 11 includes a flexible insert section 16 to be inserted into a subject, a handling section 17, and a universal cord 18. The handling section 17 is connected to a base portion of the insert section 16 and is used for holding the electronic endoscope 11 and operating the insert section 16. The universal cord 18 connects the handling section 17 to each of the processing device 12 and the light source device 13. A distal portion 16a, provided to a distal end of the insert section 16, incorporates an image sensor (not shown), an imaging optical device 19 having an objective optical system, a pair of lighting optical devices 20 (see Fig. 2). A proximal end of the distal portion 16a is connected to a flexible bending portion 16b.

The handling section 17 is provided with an angle knob 22, an operation button 23, and a forceps inlet 24. The angle knob 22 is rotated to adjust a direction and an amount of bending of the insert section 16. The operation button 23 is used for controlling various operations, such as feeding air or water and suction. The forceps inlet 24 is connected to a forceps channel extending throughout the insert section 16. An air/water channel, a signal cable, and the like extend throughout the universal cord 18.

The processing device 12 is electrically connected to the electronic endoscope 11 and the light source device 13 to control the operation of the entire electronic endoscope system 10. The processing device 12 controls the image sensor in the distal portion 16a via the signal cable extending through the universal cord 18 and the insert section 16. The processing device 12 obtains an imaging signal outputted from the image sensor via the signal cable and performs various image processes to the imaging signal. Thereby, the processing device 12 displays the imaging signal as an observation image.

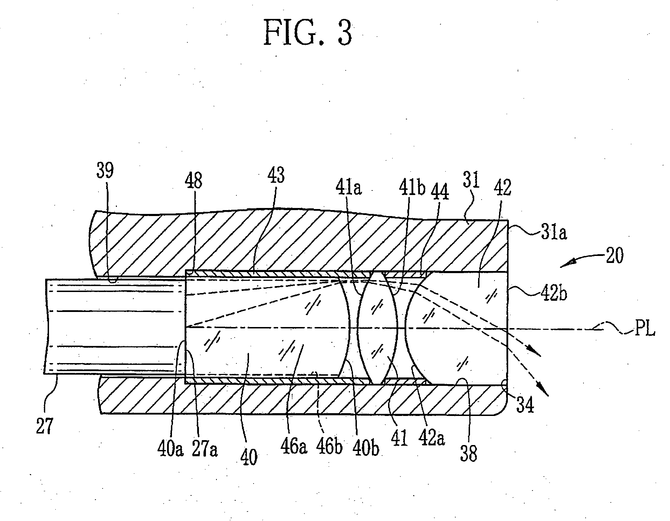

The light source device 13 with a white light source supplies illumination light to the endoscope 11. The illumination light from the light source device 13 is guided to the lighting optical device 20 through a light guide 27 (see Fig. 3). Then, the lighting optical device 20 applies the illumination light to a target (not shown). The light guide 27 is composed of a plurality of optical fibers bundled into a cylindrical shape, for example. The light guide 27 extends through the universal cord 18 and the insert section 16 such that an exit end of the light guide 27 contacts the distal portion 16a. An end surface (hereinafter referred to as a light guide incident surface, not shown) of the light guide 27, inserted into the light source device 13, faces the light source in the light source device 13. The illumination light from the light source is incident on the light guide incident surface and then comes out of the other end (hereinafter referred to as the light guide exit surface) 27a (see Fig. 3).

As shown in Fig. 2, the distal portion 16a is composed of a cylindrical distal portion body 31, the imaging optical device 19, the pair of lighting optical devices 20, and the like. The distal portion body 31 is formed of a hard resin or a metal material. An imaging window 33, a pair of illumination windows 34, an air/water nozzle 35, and a forceps outlet 36 of a forceps channel are provided on a front end surface 31a of the distal portion body 31.

The imaging window 33 is used for imaging the target and incorporates the imaging optical device 19. The imaging optical device 19 is provided with the objective optical system and an image sensor, as is well known. The image sensor images the target.

The illumination window 34 is used for illuminating the target. The lighting optical device 20 is positioned inside the illumination window 34. The lighting optical device 20 distributes the illumination light guided by the light guide 27 in a suitable manner, for example, such that the illumination light illuminates an imaging field uniformly. The imaging window 33 is provided between the pair of illumination windows 34 by way of example. The number and the arrangement of the illumination windows 34 may be changed as necessary.

The lighting optical devices 20 are inserted into respective lens mounting holes 38 formed inside the distal portion body 31. Each lens mounting hole 38 extends from the front end surface 31a toward the rear along an optical axis PL of the lighting optical device 20. An opening on the front end surface 31a is the illumination window 34. As shown in Fig. 3, behind the lens mounting hole 38 inside the distal portion body 31, a light guide mounting hole 39 is connected to the lens mounting hole 38. The light guide 27 is fitted into the light guide mounting hole 39. Each of the lens mounting hole 38 and the light guide mounting hole 39 has a circular cross section. The lens mounting hole 38 is coaxial with the light guide mounting hole 39.

The lighting optical device 20 includes an optical element 40, a first lens 41, a second lens 42, a lens barrel 43, and a spacer ring 44. The lens barrel (cylindrical lens holder) 43 in which the optical element 40 is fixed, the first lens 41, the spacer ring 44, and the second lens 42 are inserted and fixed in this order in the lens mounting hole 38.

As shown in Fig. 3, the optical element 40 is an approximately cylindrical rod lens with a core 46a, being a central member, and a clad 46b surrounding the core 46a. The refractive index of the clad 46b is lower than that of the core 46a. The optical element 40 has a planar incident surface 40a on the light guide side and a convex exit surface 40b on the illumination window side. The optical element 40 as a whole functions as the convex lens.

Because there is a difference between the refractive indices of the core 46a and the clad 46b, the light incident from the incident surface 40a is reflected inside the optical element 40 to be guided to the exit surface 40b side. In other words, an outer circumferential portion of the optical element 40 has light reflection function. Note that the optical element 40 may be composed of two or more members to exhibit the light reflection function. For example, a reflection layer may be formed around an outer circumferential surface of the rod-like plano-convex lens.

The optical element 40 is held inside the lens barrel 43. The lens barrel 43 also functions as a spacer ring for positioning the first lens 41 at a predetermined distance away from the optical element 40. An inner diameter of the lens barrel 43 is equivalent to an outer diameter of the optical element 40. An outer diameter of the lens barrel 43 is equivalent to an inner diameter of the lens mounting hole 38. The optical element 40 is fixed inside the lens barrel 43 such that the incident surface 40a of the optical element 40 is approximately flush with an edge (rear edge) of the lens barrel 43 on the light guide side.

A hole or a slot 43a (see Fig. 2) is formed on an outer circumferential surface of the lens barrel 43. For example, thermosetting resin is dropped to the hole 43a in a state that the optical element 40 is fitted inside the lens barrel 43, and then hardened. Thus, the optical element 40 is fixed inside the lens barrel 43.

An inner diameter of the light guide mounting hole 39 is slightly smaller than the inner diameter of the lens mounting hole 38 by the approximate thickness of the lens barrel 43 (for example, of the order of 0.1 mm). By coaxially connecting the lens mounting hole 38 and the light guide mounting hole 39 with different diameters, a contact surface 48 is formed to surround a circumference of the light guide mounting hole 39 at the boundary between the lens mounting hole 38 and the light guide mounting hole 39. The contact surface 48 contacts and holds the rear edge of the lens barrel 43. This determines the positions of the lens barrel 43 and the optical element 40 fixed inside the lens barrel 43. The lens barrel 43 is fixed in the lens mounting hole 38 by applying the thermosetting resin on the outer circumferential surface of the lens barrel 43, for example.

The outer diameter of the light guide 27 is approximately equivalent to the inner diameter of the light guide mounting hole 39 and the outer diameter of the optical element 40. The light guide 27 is fitted and fixed in the light guide mounting hole 39 with the light guide exit surface 27a in tightly contact with the incident surface 40a of the optical element 40. Thereby, all the illumination light from the light guide exit surface 27a is incident on the incident surface 40a of the optical element 40 without the increase in the outer diameter of the optical element 40.

Because the thickness of the clad 46b is extremely small, the diameter of the core 46a is slightly smaller than the outer diameter of the optical element. The light is incident on the core 46a being a light incident region. In this embodiment, to substantially match the outer diameter of the light guide 27 with the diameter of the core 46a, the outer diameter of the light guide 27 is slightly smaller than the outer diameter of the optical element 40.

The first lens 41 is a biconvex lens with a convex incident surface 41a and a convex exit surface 41b. An outer diameter of the first lens 41 is approximately equivalent to the inner diameter of the lens mounting hole 38 and greater than the outer diameter of the optical element 40. The first lens 41 is inserted into the lens mounting hole 38 to a position where the first lens 41 comes in contact with an edge (front edge) on the exit side of the lens barrel 43 while the outer circumference of the first lens 41 is held by the inner surface of the lens mounting hole 38. Thereby, the first lens 41 is fixed at a position at a predetermined distance away from the optical element 40. The length of the lens barrel 43 is determined based on the length of the optical element 40, a design space between the optical element 40 and the first lens 41, for example.

The inner diameter of the lens barrel 43 is approximately equivalent to the outer diameter of the optical element 40. Accordingly, a diameter of a space between the optical element 40 and the first lens 41 (that is, the inner diameter of the lens barrel 43 in this embodiment) is approximately equivalent to the outer diameter of the optical element 40. This allows the illumination light from a peripheral portion of the exit surface 40b of the optical element 40 to be incident on the first lens 41 without being obstructed. Note that the inner diameter of a portion of the lens barrel 43 between the optical element 40 and the first lens 41 may be greater than the outer diameter of the optical element 40. However, this is not preferable in view of reducing the outer diameter of the insert section 16.

Inner and outer diameters of the spacer ring 44 are equivalent to those of the lens barrel 43, for example. The spacer ring 44 determines a space between the first and second lenses 41 and 42. The spacer ring 44 is disposed between the first and second lenses 41 and 42.

The second lens 42 is a plano-convex lens with a convex incident surface 42a protruding toward the first lens 41 and a planar exit surface 42b. The exit surface 42b constitutes the illumination window 34. An outer diameter of the second lens 42 is equivalent to the inner diameter of the lens mounting hole 38. The second lens 42 is fitted into the lens mounting hole 38 such that the planar exit surface 42b is substantially flush with an end (front end) of the lens mounting hole 38.

The second lens 42 is fixed in the lens mounting hole 38, with the first lens 41 and the spacer ring 44 held between the lens barrel 43 and the second lens 42. To fix the second lens 42, for example, the thermosetting resin between the outer circumference of the second lens 42 and the inner surface of the lens mounting hole 38 is hardened. Fixing the lens barrel 43 and the second lens 42 in the lens mounting hole 38 fixes the first lens 41 and the spacer ring 44 held between the lens barrel 43 and the second lens 42.

To apply the thermosetting resin on the outer circumferential surface of the lens barrel 43, the outer circumference of the second lens 42, and an inner surface of the lens mounting hole 38, for example, each of the outer diameter of the lens barrel 43 and the outer diameter of the second lens 42 is slightly smaller (for example, of the order of 10 µm) than the inner diameter of the lens mounting hole 38. Note that groove(s) to which the thermoplastic resin is applied may be formed on the outer circumferential surface of the lens barrel 43, the outer circumference of the second lens 42, or on the inner surface of the lens mounting hole 38.

Each of the outer diameter of the first lens 41 and the outer diameter of the second lens 42 is greater than the outer diameter of the lens barrel 43 with the optical element 40. However, because the outer circumferences of the first and second lenses 41 and 42 are directly held by the inner surface of the lens mounting hole 38, the outer diameter of the distal portion 16a does not increase even if the first and second lenses 41 and 42 with the large diameters are used.

According to the above configuration, when the light source device 13 is turned on, the illumination light from the light source device 13 is incident on the light guide incident surface of the light guide 27. The illumination light is guided through the light guide 27 to the distal portion 16a and then comes out of the light guide exit surface 27a.

The illumination light from the light guide exit surface 27a is incident on the optical element 40. This illumination light includes light rays traveling in different directions in accordance with a numerical aperture of the light guide 27. Out of the light rays, those parallel with the optical axis PL are converged and then spread inside lighting optical device 20. Thereafter, the illumination light is applied to the target.

On the other hand, as shown by broken lines in Fig. 3, there are light rays traveling in directions non-parallel with the optical axis PL. Such light rays comes out of the peripheral portion of the exit surface 40b of the optical element 40 and then travel approximately parallel with the optical axis PL. The diameter of the space between the optical element 40 and the first lens 41 is equivalent to the outer diameter of the optical element 40, and there is no obstruction in this space to which the light rays are projected from the optical element 40 in the optical axis direction. Accordingly, the light rays, coming out of the peripheral portion of the exit surface 40b and then traveling approximately parallel with the optical axis PL, are incident on the first lens 41 without obstruction. Then the light rays are applied as the illumination light to the target through the second lens 42.

Accordingly, the illumination light with a large light quantity is applied to the target. In addition, light quantity loss of the illumination light inside the lighting optical device 20 decreases, which reduces heat generation caused by the light quantity loss. As a result, the temperature rise of the distal portion 16a is reduced.

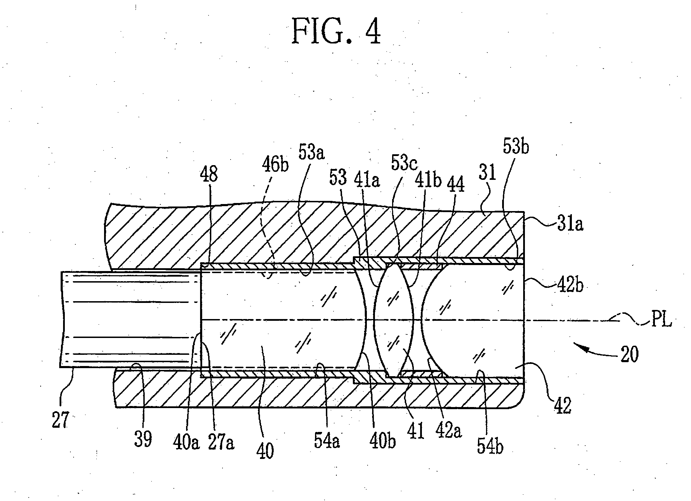

An embodiment shown in Fig. 4 is similar to that shown in Fig. 3 except that the length of a lens barrel 53 is approximately equivalent to that of a lens mounting hole 54 composed of lens mounting holes 54a and 54b. Other than that, like numerals designate like parts as in Fig. 3, and descriptions thereof are omitted.

The light guide mounting hole 39 and the lens mounting holes 54a and 54b are formed in the distal portion body 31. An outer diameter of the lens mounting hole 54b is greater than an outer diameter of the lens mounting hole 54a. Note that the lens mounting holes 54a and 54b may constitute a single hole with a constant outer diameter.

The optical element 40, the first lens 41, and the second lens 42 are fixed inside the lens barrel 53. The lens barrel 53 is fitted and fixed into the lens mounting holes 54a and 54b. The lens barrel 53 has a small diameter portion 53a for holding the optical element 40 and a large diameter portion 53b for holding the first and second lenses 41 and 42. In the lens barrel 53, a diameter of a space between the optical element 40 and the first lens 41 (that is, an inner diameter of the small diameter portion 53a) is approximately equivalent with the outer diameter of the optical element 40. This allows the light rays, coming out of the peripheral portion of the exit surface 40b and then traveling approximately parallel with the optical axis PL, to be incident on the first lens 41 without obstruction. The outer diameter of the spacer ring 44 is equivalent to each of the outer diameters of the first and second lenses 41 and 42. Note that the first lens 41 is held by a shoulder portion 53c of the lens barrel 53 and the spacer ring 44.

According to this embodiment, due to the large diameter portion 53b for holding the first and second lenses 41 and 42, the outer diameter of the lens barrel 53 is greater than that shown in Fig. 3. Accordingly, the distal potion 16a needs larger room for the lens barrel 53. However, there is an advantage that the lighting optical device 20 can be made into a unit. This facilitates assembly and replacement, for example.

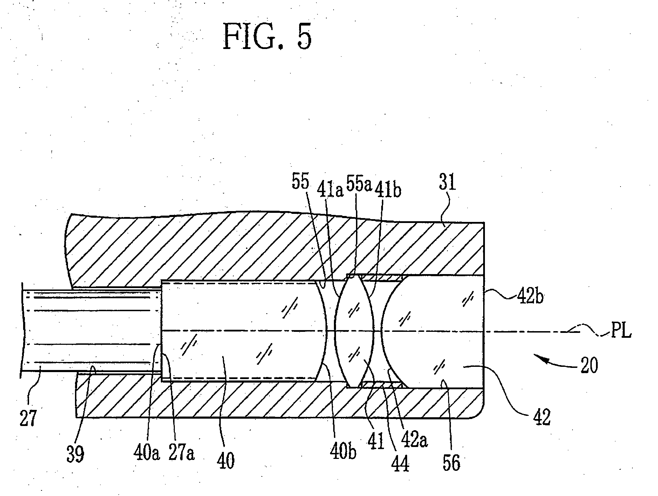

In an embodiment shown in Fig. 5, which is not covered by the claims, the optical element 40, the first and second lenses 41 and 42 are fitted into a lens mounting hole without using a lens barrel. Other than that, like numerals designate like parts as in Fig. 3, and descriptions thereof are omitted.

In this embodiment, the lens mounting hole has a small diameter portion 55 and a large diameter portion 56. The optical element 40 is fitted into the small diameter portion 55. The first and second lenses 41 and 42 are fitted into the large diameter portion 56. Accordingly, an inner diameter of the small diameter portion 55 is equivalent to the outer diameter of the optical element 40. An inner diameter of the large diameter portion 56 is equivalent to each of the outer diameters of the first and second lenses 41 and 42. A diameter of a space between the optical element 40 and the first lens 41 (that is, the inner diameter of the small diameter portion 55) is equivalent to the outer diameter of the optical element 40. Light rays, coming out of the peripheral portion of the exit surface 40b and then traveling parallel with the optical axis PL, are incident on the first lens 41 without obstruction.

The first lens 41 contacts a shoulder portion 55a formed at a boundary between the small diameter portion 55 and the large diameter portion 56. This determines the position of the first lens 41 at a predetermined distance away from the optical element 40.

According to this embodiment, to fix the optical element 40 inside the small diameter portion 55 of the lens mounting hole, the inner diameter of the light guide mounting hole 39 needs to be smaller than the outer diameter of the optical element 40. The outer diameter of the light guide 27 is restricted by the inner diameter of the light guide mounting hole 39. Accordingly, the outer diameter of the light guide 27 needs to be smaller than the outer diameter of the optical element 40. Although this embodiment cannot utilize the diameter size of the optical element 40 efficiently, the omission of the lens barrel is advantageous in view of reducing the number of parts and space.

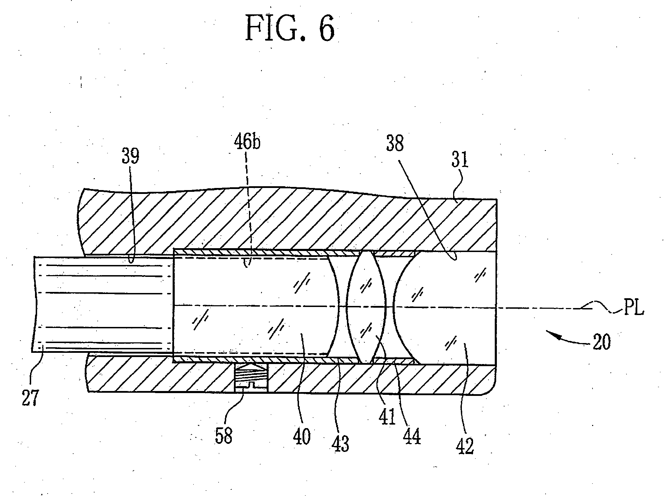

In the above embodiments, the thermosetting resin is used for fixing the lens barrel to the lens mounting hole and for fixing the optical element or each of the lenses to the lens barrel. Instead of the thermosetting resin, a UV curable resin or a common adhesive may be used. Alternatively, as shown in Fig. 6, the lens barrel 43 may be fixed inside the lens mounting hole 38 in the distal portion body 31 with a screw 58.

In the above embodiments, two lenses, being a lens optical system, are used by way of example. Alternatively, for example, a single lens or three or more lenses may be used. When the lighting optical device is composed of the optical element and a single lens, the single lens may be an aspheric lens so as to obtain a suitable light distribution property. Alternatively, a plano-convex lens with a planar incident surface and a convex exit surface, disclosed in the Japanese Patent Laid-Open Publication No. 2002-182126, may be used as the first lens.

Various changes and modifications are possible in the present invention and may be understood to be within the present invention.

## Claims

1. An endoscope (11) having a lighting optical device (20) adapted to apply illumination light from a distal portion (16a) of an insert section (16) to a target in a subject, the illumination light being transmitted from a light source to the distal portion through a light guide (27), the lighting optical device comprising:
a lens mounting hole (38) formed in a distal portion body (31), the distal portion body constituting the distal portion;
a lens barrel (43, 53) fitted into the lens mounting hole;
an optical element (40) held inside the lens barrel, the optical element having an incident surface (40a) facing the light guide, and a convex exit surface (40b);
a first lens (41) housed in the lens mounting hole, an outer diameter of the first lens being greater than an outer diameter of the optical element, the first lens being disposed in front of the optical element; and
a space between the optical element and the first lens, a diameter of the space being greater than or equal to the outer diameter of the optical element,
**characterized in that**
an outer circumferential portion (46b) of the optical element (40) has a light reflection layer, and the first lens (41) is a biconvex lens and is fixed at a predetermined distance away from the optical element (40).

2. The endoscope of claim 1, wherein the lighting optical device further includes a light guide mounting hole (39), formed inside the distal portion body, for housing the light guide, and the light guide mounting hole is connected to the lens mounting hole, and an outer diameter of the light guide mounting hole is smaller than an outer diameter of the lens mounting hole.

3. The endoscope of claim 2, wherein the outer diameter of the optical element is approximately equivalent to an outer diameter of the light guide.

4. The endoscope of claim 2, wherein the lighting optical device further includes a second lens (42) disposed in front of the first lens inside the lens mounting hole, and an outer diameter of the second lens is equivalent to the outer diameter of the first lens.

5. The endoscope of claim 4, wherein the incident surface of the optical element is planar and approximately flush with a rear edge of the lens barrel.

6. The endoscope of claim 5, wherein the optical element is a rod lens including a core (46a) and a clad (46b) surrounding the core, and a refractive index of the clad is lower than a refractive index of the core.

7. The endoscope of claim 6, further including a spacer ring (44) disposed between the first and second lenses to keep a distance between the first and second lenses.

8. The endoscope of claim 7, wherein the lens barrel is shorter than the lens mounting hole, and a front edge of the lens barrel is positioned inside the lens mounting hole, and the first lens contacts the front edge of the lens barrel, and the first lens, the spacer ring, and the second lens fit into the lens mounting hole.

9. The endoscope of claim 7, wherein a length of the lens barrel is equivalent with a length of the lens mounting hole, and the lens barrel has a small diameter portion (53a), a large diameter portion (53b), and a shoulder portion (53c) formed between the small and large diameter portions, and the optical element is fitted into the small diameter portion, and the first lens contacts the shoulder portion, and the first lens, the spacer ring, and the second lens are fitted into the large diameter portion.

## Patentansprüche

1. Endoskop (11) mit einer Beleuchtungsoptikeinrichtung (20), ausgebildet zum Aufbringen von Beleuchtungslicht aus einem distalen Abschnitt (16a) eines Einführteils (16) auf ein Target in einem Subjekt, wobei das Beleuchtungslicht von einer Lichtquelle durch einen Lichtleiter (27) zu dem distalen Abschnitt gelangt, umfassend:
ein Linsenaufnahmeloch (38), das in einem distalen Körperabschnitt (31) gebildet ist, der den distalen Abschnitt darstellt;
eine Linsenhülse (43, 53), die in das Linsenaufnahmeloch eingepasst ist;
ein optisches Element (40), das im Inneren der Linsenhülse gehalten ist, wobei das optische Element eine dem Lichtleiter gegenüber liegende Einfallfläche (40a) und eine konvexe Austrittsfläche (40b) besitzt;
eine erste Linse (41), die in dem Linsenaufnahmeloch untergebracht ist, wobei ein Außendurchmesser der ersten Linse größer ist als ein Außendurchmesser des optischen Elements und die erste Linse sich vor dem optischen Element befindet; und
einen Raum zwischen dem optischen Element und der ersten Linse, wobei ein Durchmesser des Raums größer oder gleich ist dem Außendurchmesser des optischen Elements,
**dadurch gekennzeichnet, dass**
ein Außenumfangsabschnitt (46b) des optischen Elements (40) eine Lichtreflexionsschicht besitzt, und die erste Linse (49) eine bikonvexe Linse ist und in einem vorbestimmten Abstand entfernt von dem optischen Element (40) fixiert ist.

2. Endoskop nach Anspruch 1, bei dem die Beleuchtungsoptikeinrichtung weiterhin ein Lichtleiteraufnahmeloch (39) enthält, ausgebildet im Innern des distalen Körperabschnitts um den Lichtleiter aufzunehmen, und das Lichtleiteraufnahmeloch mit dem Linsenaufnahmeloch verbunden ist, und ein Außendurchmesser des Lichtleiteraufnahmelochs kleiner ist als ein Außendurchmesser des Linsenaufnahmelochs.

3. Endoskop nach Anspruch 2, bei dem der Außendurchmesser des optischen Elements nahezu äquivalent dem Außendurchmesser des Lichtleiters ist.

4. Endoskop nach Anspruch 2, bei dem die Beleuchtungsoptikeinrichtung weiterhin eine zweite Linse (42) vor der ersten Linse im Innern des Linsenaufnahmelochs enthält, und ein Außendurchmesser der zweiten Linse äquivalent ist zum Außendurchmesser der ersten Linse.

5. Endoskop nach Anspruch 4, bei dem die Einfallfläche des optischen Elements planar und nahezu bündig mit einer hinteren Kante der Linsenhülse ist.

6. Endoskop nach Anspruch 5, bei dem das optische Element eine Stablinse mit einem Kern (46a) und einem den Kern umgebenden Mantel (46b) ist, wobei ein Brechungsindex des Mantels geringer ist als ein Brechungsindex des Kerns.

7. Endoskop nach Anspruch 6, weiterhin enthaltend einen Distanzring (44) zwischen der ersten und der zweiten Linse, um einen Abstand zwischen der ersten und der zweiten Linse zu halten.

8. Endoskop nach Anspruch 7, bei der die Linsenhülse kürzer ist als das Linsenaufnahmeloch, und eine Vorderkante der Linsenhülse im Inneren des Linsenaufnahmeloch positioniert ist, wobei die erste Linse die vordere Kante der Linsenhülse berührt, und die erste Linse, der Distanzring und die zweite Linse in das Linsenaufnahmeloch passen.

9. Endoskop nach Anspruch 7, bei dem eine Länge der Linsenhülse äquivalent ist zu einer Länge des Linsenaufnahmelochs, und die Linsenhülse einen Abschnitt (53a) kleinen Durchmessers, einen Abschnitt (53b) großen Durchmessers und einen zwischen den Abschnitten kleinen und großen Durchmessers gebildeten Schulterabschnitt (43c) besitzt, und das optische Element in den Abschnitt kleinen Durchmessers eingepasst ist, und die erste Linse den Schulterabschnitt berührt, ferner die erste Linse, der Distanzring und die zweite Linse in den Abschnitt großen Durchmessers eingepasst sind.

## Revendications

1. Endoscope (11) présentant un dispositif optique d'éclairage (20) apte à appliquer une lumière d'éclairage d'une partie distale (16a) d'une section d'introduction (16) à une cible dans un sujet, la lumière d'éclairage étant transmise d'une source lumineuse à la partie distale par le biais d'un conduit de lumière (27), le dispositif optique d'éclairage comprenant :
un trou de monture de lentille (38) formé dans un corps de partie distale (31), le corps de partie distale constituant la partie distale ;
un barillet de lentille (43, 53) monté dans le trou de monture de lentille ;
un élément optique (40) maintenu à l'intérieur du barillet de lentille, l'élément optique présentant une surface incidente (40a) faisant face au conduit de lumière, et une surface de sortie convexe (40b) ;
une première lentille (41) logée dans le trou de monture de lentille, un diamètre extérieur de la première lentille étant supérieur à un diamètre extérieur de l'élément optique, la première lentille étant disposée devant l'élément optique, et
un espace entre l'élément optique et la première lentille, un diamètre de l'espace étant supérieur ou égal au diamètre extérieur de l'élément optique,
**caractérisé en ce que**
une partie circonférentielle extérieure (46b) de l'élément optique (40) présente une couche de réflexion de la lumière, et la première lentille (41) est une lentille biconvexe et est fixée à une distance prédéterminé éloignée de l'élément optique (40).

2. Endoscope selon la revendication 1, dans lequel le dispositif optique d'éclairage inclut en outre un trou de monture de conduit de lumière (39), formé à l'intérieur du corps de partie distale, destiné à loger le conduit de lumière, et le trou de monture de conduit de lumière est relié au trou de monture de lentille, et un diamètre extérieur du trou de monture de conduit de lumière est inférieur à un diamètre extérieur du trou de monture de lentille.

3. Endoscope selon la revendication 2, dans lequel le diamètre extérieur de l'élément optique est approximativement équivalent à un diamètre extérieur du conduit de lumière.

4. Endoscope selon la revendication 2, dans lequel le dispositif optique d'éclairage inclut en outre une seconde lentille (42) disposée devant la première lentille à l'intérieur du trou de monture de lentille, et un diamètre extérieur de la seconde lentille est équivalent au diamètre extérieur de la première lentille.

5. Endoscope selon la revendication 4, dans lequel la surface incidente de l'élément optique est plane et approximativement à ras d'un bord arrière du barillet de lentille.

6. Endoscope selon la revendication 5, dans lequel l'élément optique est une lentille cylindrique incluant un coeur (46a) et une gaine (46b) entourant le coeur, et un indice de réfraction de la gaine est inférieur à un indice de réfraction du coeur.

7. Endoscope selon la revendication 6, incluant en outre un anneau entretoise (44) disposé entre les première et seconde lentilles afin de conserver une distance entre les première et seconde lentilles.

8. Endoscope selon la revendication 7, dans lequel le barillet de lentille est plus court que le trou de monture de lentille, et un bord avant du barillet de lentille est positionné à l'intérieur du trou de monture de lentille, et la première lentille est en contact avec le bord avant du barillet de lentille, et la première lentille, l'anneau entretoise et la seconde lentille tiennent dans le trou de monture de lentille.

9. Endoscope selon la revendication 7, dans lequel une longueur du barillet de lentille est équivalente à une longueur du trou de monture de lentille, et le barillet de lentille présente une partie de petit diamètre (53a), une partie de grand diamètre (53b), et une partie d'épaulement (53c) formée entre les parties de petit et de grand diamètres ; l'élément optique est monté dans la partie de petit diamètre, et la première lentille est en contact avec la partie d'épaulement, et la première lentille, l'anneau entretoise et la seconde lentille sont montés dans le trou de monture de lentille.
